# EUROPEAN PATENT SPECIFICATION

(11) **EP 4 261 278 B1**
(45) Date of publication and mention of the grant of the patent: **12.11.2025**
(21) Application number: 22871087.7
(22) Date of filing: 30.12.2022
(51) Int. Cl.: C12N 5/20, G01N 33/569, C07K 16/40, C07K 16/00, C12N 5/16, G01N 33/58, C07K 16/12, C12Q 1/04, G01N 33/543, C12Q 1/25

(54) **ANTI-NEW DELHI METALLO-BETA-LACTAMASE (NDM)-TYPE CARBAPENEMASE MOUSE HYBRIDOMA CELL LINE, MONOCLONAL ANTIBODY (mAb), AND APPLICATION THEREOF**
ANTI-NEUE DELHI-METALLO-BETA-LACTAMASE (NDM)-TYP-CARBAPENEMASE-MAUS-HYBRIDOMA-ZELLLINIE, MONOKLONALER ANTIKÖRPER (mAb) UND ANWENDUNG DAVON
LIGNÉE CELLULAIRE D'HYBRIDOME DE SOURIS EXPRIMANT UN ANTICORPS CONTRE UNE CARBAPENÉMASE DE TYPE NEW DELHI METALLO-BÊTA-LACTAMASE (NDM), ANTICORPS MONOCLONAL (mAb), ET SON APPLICATION

(30) Priority: 02.03.2022 CN 202210194930; 13.10.2022 CN 202211253434
(43) Date of publication of application: 18.10.2023
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN); Genobio Pharmaceutical Co., Ltd., Binhai New Area Tianjin 300480 (CN)
(72) Inventor: YUAN, Qinghua, Tianjin 300480 (CN); LI, Keke, Tianjin 300480 (CN); HE, Yongsheng, Tianjin 300480 (CN); CHEN, Xiaoling, Tianjin 300480 (CN); WANG, Yamiao, Tianjin 300480 (CN); KONG, Di, Tianjin 300480 (CN); WANG, Siyi, Tianjin 300480 (CN); ZHOU, Yuehui, Tianjin 300480 (CN)
(74) Representative: Bayramoglu et al.
(86) International application number: PCT/CN2022/143804
(87) International publication number: WO 2023/165252

(56) References cited:
- WO-A1-2016/092096
- WO-A1-2018/106546
- CN-A- 107 667 291
- CN-A- 111 273 040
- CN-A- 112 501 131
- CN-A- 112 980 803
- CN-A- 113 249 336
- CN-A- 114 316 056
- CN-A- 114 755 420
- JP-A- 2019 142 811
- US-A1- 2012 219 952

## Description

### TECHNICAL FIELD

The present disclosure belongs to the technical field of antibody preparation, and in particular relates to an anti-New Delhi metallo-β-lactamase (NDM)-type carbapenemase mouse hybridoma cell line, a monoclonal antibody (mAb), and an application thereof.

### BACKGROUND

Carbapenems are one of the most effective drugs for controlling clinical pathogen infections. Carbapenemase-producing organisms (CPOs) and carbapenem-resistant *Enterobacteriaceae* (CRE) have become a global public health issue due to their broad-spectrum drug resistance and related patients have very limited treatment options. Carbapenemases refer to a group of beta-lactamases that can significantly hydrolyze at least imipenem (IMP) or meropenem (MEM), including class A, B, and D enzymes based on Ambler molecular structure classification. The class B enzymes refer to Metallo-β-lactamases (IMP type, verona integron-encoded metallo-β-lactamase (VIM) type, NDM type, or the like), which are referred to as metalloenzymes and are mainly present in *Pseudomonas aeruginosa* (*P. aeruginosa*), *Acinetobacter,* and *Enterobacteriaceae* bacteria. The class A and D enzymes refer to serinases, the class A enzymes (*Klebsiella pneumoniae* (*K. pneumoniae*) carbapenemase (KPC) type or the like) are mainly present in *Enterobacteriaceae* bacteria, and the class D enzymes (oxacillin-hydrolysing (OXA) type) are mainly present in *Acinetobacter.*

Since NDM was first found in a *K. pneumoniae* strain isolated from a Swedish patient who had traveled in India in 2008, NDM has spread worldwide at an alarming rate. So far, NDM has appeared in dozens of countries in Europe, the United States, Canada, and Mexico in North America, and China, Japan, South Korea, and Singapore in Asia. In India, Pakistan, or the like, NDM has been prevalent, with a detection rate of 38.5%.

There are numerous methods for laboratory detection of carbapenemases. The methods mainly include modified Hodge test, Carba NP test, modified carbapenem inactivation method (mCIM), enzyme inhibitor enhancement test, immunogold labeling test, molecular biotechnology, and the like. Immunodiagnostic reagents are the fastest-growing segment of *in vitro* diagnostic reagents, and achieve qualitative or quantitative detection through specific binding between an antigen and an antibody. Carbapenemase rapid diagnostic products play a prominent role in the early typing and medication guidance for drug-resistant strains, and the research and development of carbapenemase rapid diagnostic products is still imminent. JP2019142811A discloses a monoclonal antibody for detecting a New Delhi metallo β-lactamase (NDM type MBL) -producing bacterium using an immunochromatography method and also in Colloidal gold-labelled methods.

### SUMMARY

In order to solve the above technical problems, the present disclosure provides an anti-NDM-type carbapenemase mouse mAb, and an application thereof.

The present disclosure adopts the following technical solutions: an anti-NDM-type carbapenemase mouse mAb is provided, which is:
an antibody 1GH10, including a light chain variable region and a heavy chain variable region, where the light chain variable region includes CDRL1 as shown in SEQ ID NO: 1, CDRL2 as shown in SEQ ID NO: 2, and CDRL3 as shown in SEQ ID NO: 3, and the heavy chain variable region includes CDRH1 as shown in SEQ ID NO: 4, CDRH2 as shown in SEQ ID NO: 5, and CDRH3 as shown in SEQ ID NO: 6; the light chain variable region of the antibody 1GH10 has an amino acid sequence as shown in SEQ ID NO: 7, and the heavy chain variable region of the antibody 1GH10 has an amino acid sequence as shown in SEQ ID NO: 9:
   SEQ ID NO: 1 RSSQSIVHSDGNTYLD (CDRL1)
   SEQ ID NO: 2 KVSNRFS (CDRL2)
   SEQ ID NO: 3 FQISRVPFT (CDRL3)
   SEQ ID NO: 4 GYAFSNYLIE (CDRH1)
   SEQ ID NO: 5 VINPGRDDTNYNEKFKG (CDRH2)
   SEQ ID NO: 6 FPSILRYDSGSFSYFGLDC (CDRH3);
   SEQ ID NO: 7 
DVLMTQTPLSLSVSLGDQASISCRSSQSIVHSDGNTYLDWYLQKPGQSPKLLIYKVSN
RFSGVPDRFSGSGSGTDFTLKITRVEAEDLGVYYCFQISRVPFTFGSGTKLEIKRA
   SEQ ID NO: 9 
SRDYGLRVELVRPGTSVKVSCKASGYAFSNYLIEWVKQRPGQGLEWIGVINPGRDDTN
YNEKFKGKATLTADKSSSTAYMHLSSLTSDDSAVYFCARFPSILRYDSGSFSYFGLDCWGQG
TSVTVSSA;
   or
an antibody 12HE11, including a light chain variable region and a heavy chain variable region, where the light chain variable region includes CDRL1 as shown in SEQ ID NO: 11, CDRL2 as shown in SEQ ID NO: 12, and CDRL3 as shown in SEQ ID NO: 13, and the heavy chain variable region includes CDRH1 as shown in SEQ ID NO: 14, CDRH2 as shown in SEQ ID NO: 15, and CDRH3 as shown in SEQ ID NO: 16; the light chain variable region of the antibody 12HE11 has an amino acid sequence as shown in SEQ ID NO: 17, and the heavy chain variable region of the antibody 12HE11 has an amino acid sequence as shown in SEQ ID NO: 19:
   SEQ ID NO: 11 RSSQSLFNSGNQKNYLT (CDRL1)
   SEQ ID NO: 12 WAVTRES (CDRL2)
   SEQ ID NO: 13 QNDYSYPLT (CDRL3)
   SEQ ID NO: 14 GYTLSDYHVK (CDRH1)
   SEQ ID NO: 15 DIRPQNGDIVYNQKFKD (CDRH2)
   SEQ ID NO: 16 HYYAYGKWFPY (CDRH3)
   SEQ ID NO: 17 
DILMTQSPSSLTVTAGEKVTLSCRSSQSLFNSGNQKNYLTWHQQKPGQSPKLLIYWAV
TRESGVPDRFTGSGSGTDFTLTISSVQAEDLAIYYCQNDYSYPLTFGAGTKLELK
   SEQ ID NO: 19

VQLHESGPELVKPGASVKLSCTASGYTLSDYHVKWVKQNHGQSLEWIGDIRPQNGDI
VYNQKFKDKATLTVDKSSTTAYIRLSSLTSEDSAVYYCAFHYYAYGKWFPYWGQGTLVTVS

Preferably, the antibody 1GH10 is produced by an anti-NDM-type carbapenemase mouse hybridoma cell line with an accession number of CGMCC No. 21963; and
the antibody 12HE11 is produced by an anti-NDM-type carbapenemase mouse hybridoma cell line with an accession number of CGMCC No. 21964.

A nucleic acid molecule is provided, including a nucleotide encoding the anti-NDM-type carbapenemase mouse mAb.

Preferably, a nucleotide sequence of the nucleic acid molecule encoding the light chain variable region of the antibody 1GH10 is shown in SEQ ID NO: 8, and a nucleotide sequence of the nucleic acid molecule encoding the heavy chain variable region of the antibody 1GH10 is shown in SEQ ID NO: 10:
SEQ ID NO: 8 
GATGTTTTGATGACCCAAACTCCACTCTCCCTGTCTGTCAGTCTTGGAGATCAAGCC
TCCATCTCTTGCAGATCTAGTCAGAGCATTGTACATAGTGATGGAAACACCTATTTAGAC
TGGTATCTGCAGAAACCAGGCCAGTCTCCAAAACTCCTAATCTACAAAGTTTCCAACCG
ATTTTCTGGGGTCCCAGACAGGTTCAGTGGCAGTGGATCAGGGACAGATTTCACACTCA
AGATCACCAGAGTGGAGGCTGAGGATCTGGGAGTTTATTACTGCTTTCAAATTTCACGT
GTTCCATTCACGTTCGGCTCGGGGACAAAGTTGGAAATAAAACGGGCT
SEQ ID NO: 10 
TCCAGAGATTATGGACTTCGGGTTGAGCTGGTAAGGCCTGGGACTTCAGTGAAGGT
GTCCTGCAAGGCTTCTGGATACGCCTTCAGTAATTACTTGATAGAGTGGGTAAAGCAGA
GGCCTGGACAGGGCCTTGAGTGGATTGGAGTGATTAATCCTGGAAGGGATGATACTAAC
TACAATGAGAAGTTCAAGGGCAAGGCAACACTGACTGCAGACAAATCCTCCAGCACTG
CCTACATGCACCTCAGCAGTCTGACATCTGATGACTCTGCGGTCTACTTCTGTGCAAGAT
TCCCTTCTATTCTTCGCTACGATAGTGGCTCTTTTTCTTATTTTGGTTTGGACTGCTGGGG
TCAAGGAACCTCAGTCACCGTCTCTTCAGCC; and
a nucleotide sequence of the nucleic acid molecule encoding the light chain variable region of the antibody 12HE11 is shown in SEQ ID NO: 18, and a nucleotide sequence of the nucleic acid molecule encoding the heavy chain variable region of the antibody 12HE11 is shown in SEQ ID NO: 20:
   SEQ ID NO: 18 
GACATTCTGATGACACAGTCTCCCTCCTCCCTGACTGTGACAGCAGGCGAGAAGGT
CACTTTGAGCTGCAGGTCCAGTCAGAGTCTGTTTAACAGTGGAAATCAAAAGAACTACT
TGACCTGGCACCAGCAGAAACCAGGGCAGTCTCCTAAATTGTTGATCTACTGGGCAGTC
ACTAGGGAATCTGGGGTCCCTGATCGCTTCACAGGCAGTGGATCTGGAACAGATTTCAC
TCTCACCATCAGCAGTGTGCAGGCTGAAGACCTGGCAATTTATTACTGTCAGAATGATTA
TAGTTATCCGCTCACGTTCGGTGCTGGGACCAAACTGGAACTGAAA
   SEQ ID NO: 20

GTGCAGCTTCACGAGTCAGGACCTGAGCTGGTGAAGCCTGGGGCTTCAGTGAAAC
TGTCCTGCACGGCTTCCGGATACACCCTCAGTGACTACCACGTAAAATGGGTGAAACAG
AACCATGGACAGAGCCTTGAGTGGATTGGAGATATTAGGCCTCAGAATGGTGATATTGTT

TACAACCAGAAGTTCAAGGACAAGGCCACATTGACTGTAGACAAATCCTCCACAACAG
CCTACATACGACTCAGCAGCCTGACATCTGAGGACTCTGCAGTCTATTACTGTGCATTCC
ATTATTACGCCTACGGGAAGTGGTTTCCTTATTGGGGCCAAGGGACTCTGGTCACCGTCT
CC.

An application of the anti-NDM-type carbapenemase mouse mAb in preparation of a reagent for *in vitro* detection of an NDM-type carbapenemase antigen is provided.

Preferably, the anti-NDM-type carbapenemase mouse mAb is used to prepare an *in vitro* diagnostic kit or a microfluidic chip; and the *in vitro* diagnostic kit is a colloidal gold immunoassay kit, a chemiluminescence kit, a radioimmunoassay kit, an enzyme-linked immunoassay kit, or a fluorescence immunoassay kit.

Preferably, when the anti-NDM-type carbapenemase mouse mAb is used in preparation of a double-antibody sandwich immuno-colloidal gold test card, the antibody 1GH10 is used as a coated antibody and the antibody 12HE11 is used as a gold-labeled antibody; or
the antibody 12HE11 is used as a coated antibody and the antibody 1GH10 is used as a gold-labeled antibody.

The present disclosure has the following advantages and positive effects: the present disclosure provides two anti-NDM-type carbapenemase mouse hybridoma cell lines, which can produce two mouse anti-NDM-type carbapenemase antibodies, respectively. According to systematic evaluation including evaluation on the antibody subtype and titer and the kit sensitivity, specificity, and stability, the anti-NDM-type carbapenemase mouse mAb has excellent performance in all aspects and has a titer of 1:1,280,000 or more. Thus, the anti-NDM-type carbapenemase mouse mAb can be used as an immunodiagnostic reagent in preparation of an *in vitro* diagnostic kit for detecting carbapenemase-resistant strains.

### BRIEF DESCRIPTION OF THE DRAWINGS

FIG. 1 is a protein electrophoresis pattern of an NDM-type carbapenemase;
FIG. 2 is a protein electrophoresis pattern of anti-NDM-type carbapenemase mouse mAbs; and
FIG. 3 shows detection results of NDM-type carbapenemase test cards by a colloidal gold method.

Biological material: 1GH10 was deposited in the China General Microbiological Culture Collection Center (CGMCC) on March 24, 2021 with an accession number of CGMCC No. 21963.

Biological material: 12HE11 was deposited in the China General Microbiological Culture Collection Center (CGMCC) on March 24, 2021 with an accession number of CGMCC No. 21964.

### DETAILED DESCRIPTION OF THE EMBODIMENTS

The embodiments of the present disclosure are explained below.

The present disclosure provides an anti-NDM-type carbapenemase mouse hybridoma cell line, which is named 1GH10 and belongs to the hybridoma cell. 1GH10 was deposited in the China General Microbiological Culture Collection Center (CGMCC) on March 24, 2021 with an accession number of CGMCC No. 21963 and is tested to be alive. The present disclosure provides another anti-NDM-type carbapenemase mouse hybridoma cell line, which is named 12HE11 and belongs to the hybridoma cell. 12HE11 was deposited in the China General Microbiological Culture Collection Center (CGMCC) on March 24, 2021, with an accession number of CGMCC No. 21964 and is tested to be alive.

An antibody 1GH10 produced by an anti-NDM-type carbapenemase mouse hybridoma cell line is provided, including a light chain variable region and a heavy chain variable region, where the light chain variable region includes CDRL1 as shown in SEQ ID NO: 1, CDRL2 as shown in SEQ ID NO: 2, and CDRL3 as shown in SEQ ID NO: 3, and the heavy chain variable region includes CDRH1 as shown in SEQ ID NO: 4, CDRH2 as shown in SEQ ID NO: 5, and CDRH3, as shown in SEQ ID NO: 6:
SEQ ID NO: 1 RSSQSIVHSDGNTYLD (CDRL1)
SEQ ID NO: 2 KVSNRFS (CDRL2)
SEQ ID NO: 3 FQISRVPFT (CDRL3)
SEQ ID NO: 4 GYAFSNYLIE (CDRH1)
SEQ ID NO: 5 VINPGRDDTNYNEKFKG (CDRH2)
SEQ ID NO: 6 FPSILRYDSGSFSYFGLDC (CDRH3).

The light chain variable region of the antibody 1GH10 has an amino acid sequence as shown in SEQ ID NO: 7, and the heavy chain variable region of the antibody 1GH10 has an amino acid sequence as shown in SEQ ID NO: 9:
SEQ ID NO: 7 
DVLMTQTPLSLSVSLGDQASISCRSSQSIVHSDGNTYLDWYLQKPGQSPKLLIYKVSN
RFSGVPDRFSGSGSGTDFTLKITRVEAEDLGVYYCFQISRVPFTFGSGTKLEIKRA
SEQ **ID** NO: 9

SRDYGLRVELVRPGTSVKVSCKASGYAFSNYLIEWVKQRPGQGLEWIGVINPGRDDTN
YNEKFKGKATLTADKSSSTAYMHLSSLTSDDSAVYFCARFPSILRYDSGSFSYFGLDCWGQG
TSVTVSSA.

A nucleotide sequence encoding the light chain variable region of the antibody 1GH10 is shown in SEQ ID NO: 8, and a nucleotide sequence encoding the heavy chain variable region of the antibody 1GH10 is shown in SEQ ID NO: 10:
SEQ ID NO: 8 
GATGTTTTGATGACCCAAACTCCACTCTCCCTGTCTGTCAGTCTTGGAGATCAAGCC
TCCATCTCTTGCAGATCTAGTCAGAGCATTGTACATAGTGATGGAAACACCTATTTAGAC
TGGTATCTGCAGAAACCAGGCCAGTCTCCAAAACTCCTAATCTACAAAGTTTCCAACCG
ATTTTCTGGGGTCCCAGACAGGTTCAGTGGCAGTGGATCAGGGACAGATTTCACACTCA
AGATCACCAGAGTGGAGGCTGAGGATCTGGGAGTTTATTACTGCTTTCAAATTTCACGT
GTTCCATTCACGTTCGGCTCGGGGACAAAGTTGGAAATAAAACGGGCT
SEQ ID NO: 10

TCCAGAGATTATGGACTTCGGGTTGAGCTGGTAAGGCCTGGGACTTCAGTGAAGGT
GTCCTGCAAGGCTTCTGGATACGCCTTCAGTAATTACTTGATAGAGTGGGTAAAGCAGA
GGCCTGGACAGGGCCTTGAGTGGATTGGAGTGATTAATCCTGGAAGGGATGATACTAAC
TACAATGAGAAGTTCAAGGGCAAGGCAACACTGACTGCAGACAAATCCTCCAGCACTG
CCTACATGCACCTCAGCAGTCTGACATCTGATGACTCTGCGGTCTACTTCTGTGCAAGAT
TCCCTTCTATTCTTCGCTACGATAGTGGCTCTTTTTCTTATTTTGGTTTGGACTGCTGGGG
TCAAGGAACCTCAGTCACCGTCTCTTCAGCC.

An antibody 12HE11 produced by an anti-NDM-type carbapenemase mouse hybridoma cell line is provided, including a light chain variable region and a heavy chain variable region, where the light chain variable region includes CDRL1 as shown in SEQ ID NO: 11, CDRL2 as shown in SEQ ID NO: 12, and CDRL3 as shown in SEQ ID NO: 13, and the heavy chain variable region includes CDRH1 as shown in SEQ ID NO: 14, CDRH2 as shown in SEQ ID NO: 15, and CDRH3 as shown in SEQ ID NO: 16:
SEQ ID NO: 11 RSSQSLFNSGNQKNYLT (CDRL1)
SEQ ID NO: 12 WAVTRES (CDRL2)
SEQ ID NO: 13 QNDYSYPLT (CDRL3)
SEQ ID NO: 14 GYTLSDYHVK (CDRH1)
SEQ ID NO: 15 DIRPQNGDIVYNQKFKD (CDRH2)
SEQ ID NO: 16 HYYAYGKWFPY (CDRH3).

The light chain variable region of the antibody 12HE11 has an amino acid sequence as shown in SEQ ID NO: 17, and the heavy chain variable region of the antibody 12HE11 has an amino acid sequence as shown in SEQ ID NO: 19:
SEQ ID NO: 17 
DILMTQSPSSLTVTAGEKVTLSCRSSQSLFNSGNQKNYLTWHQQKPGQSPKLLIYWAV
TRESGVPDRFTGSGSGTDFTLTISSVQAEDLAIYYCQNDYSYPLTFGAGTKLELK
SEQ ID NO: 19

VQLHESGPELVKPGASVKLSCTASGYTLSDYHVKWVKQNHGQSLEWIGDIRPQNGDI
VYNQKFKDKATLTVDKSSTTAYIRLSSLTSEDSAVYYCAFHYYAYGKWFPYWGQGTLVTVS

A nucleotide sequence encoding the light chain variable region of the antibody 12HE11 is shown in SEQ ID NO: 18, and a nucleotide sequence encoding the heavy chain variable region of the antibody 12HE11 is shown in SEQ ID NO: 20:
SEQ ID NO: 18 
GACATTCTGATGACACAGTCTCCCTCCCTGACTGTGACAGCAGGCGAGAAGGT
CACTTTGAGCTGCAGGTCCAGTCAGAGTCTGTTTAACAGTGGAAATCAAAAGAACTACT
TGACCTGGCACCAGCAGAAACCAGGGCAGTCTCCTAAATTGTTGATCTACTGGGCAGTC
ACTAGGGAATCTGGGGTCCCTGATCGCTTCACAGGCAGTGGATCTGGAACAGATTTCAC
TCTCACCATCAGCAGTGTGCAGGCTGAAGACCTGGCAATTTATTACTGTCAGAATGATTA
TAGTTATCCGCTCACGTTCGGTGCTGGGACCAAACTGGAACTGAAA
SEQ ID NO: 20

GTGCAGCTTCACGAGTCAGGACCTGAGCTGGTGAAGCCTGGGGCTTCAGTGAAAC
TGTCCTGCACGGCTTCCGGATACACCCTCAGTGACTACCACGTAAAATGGGTGAAACAG
AACCATGGACAGAGCCTTGAGTGGATTGGAGATATTAGGCCTCAGAATGGTGATATTGTT
TACAACCAGAAGTTCAAGGACAAGGCCACATTGACTGTAGACAAATCCTCCACAACAG

CCTACATACGACTCAGCAGCCTGACATCTGAGGACTCTGCAGTCTATTACTGTGCATTCC
ATTATTACGCCTACGGGAAGTGGTTTCCTTATTGGGGCCAAGGGACTCTGGTCACCGTCT
CC.

The anti-NDM-type carbapenemase mouse mAb is subjected to systematic evaluation, including evaluation on the antibody subtype and titer and the kit sensitivity, specificity, and stability, and the anti-NDM-type carbapenemase mouse mAb has excellent performance in all aspects and thus can be used as an immunodiagnostic reagent in preparation of an *in vitro* diagnostic kit. The anti-NDM-type carbapenemase mouse mAb can be used to prepare a colloidal gold immunoassay kit, a chemiluminescence kit, a radioimmunoassay kit, an enzyme-linked immunoassay kit, or a fluorescence immunoassay kit, or can be used to prepare a microfluidic chip; and the prepared kit can detect an NDM-type carbapenemase antigen. The two antibodies involved in the present disclosure are especially suitable for preparation of a double-antibody sandwich immuno-colloidal gold test card, where the antibody 1GH10 is used as a coated antibody and the antibody 12HE11 is used as a gold-labeled antibody. The prepared double-antibody sandwich immuno-colloidal gold test card has high sensitivity. In addition, the antibody 1GH10 can be used as the gold-labeled antibody and the antibody 12HE11 can be used as the coated antibody.

The present disclosure is further explained by specific embodiments below. Specifically, the experimental methods where operation steps are not specified are carried out in accordance with the corresponding product specifications. The instruments, reagents, and consumables used in the embodiments can be purchased from commercial companies unless otherwise specified.

### Embodiment 1: Preparation of an anti-NDM-type carbapenemase mouse mAb

### 1.1 Antigen preparation

Acquisition of a recombinant plasmid: An NDM-type gene sequence was introduced into a vector Pet-28a(+), and gene synthesis was carried out.

Transformation into a host strain: the vector Pet-28a(+) carrying the target gene was transformed into a cloning host *Escherichia coli* (*E. coli*)*.*

Expanded culture: A verified positive transformant was subjected to expanded culture, then transferred to a kanamycin-resistant LB liquid medium, and cultured under shaking in a shaker at 37°C.

Induction: when an OD value of a resulting bacterial solution was 0.4 to 0.6, isopropylthio-β-galactoside (IPTG) was added at a final concentration of 0.1 mM, then bacteria were further cultured at 30°C for 4 h, and resulting bacterial cells were collected through centrifugation.

Ultrasonication of bacterial cells: the bacterial cells were resuspended by adding 30 mL of phosphate buffered saline (PBS) to about every 1 g of the bacterial cells, and then subjected to ultrasonication at a power of 400 W for about 30 minutes (ultrasonic time: 3 s, interval: 5 s); and after a resulting bacterial solution was non-viscous and clear, the bacterial solution was centrifuged to remove bacterial debris, and a resulting supernatant was filtered through a 0.45 µm filter membrane.

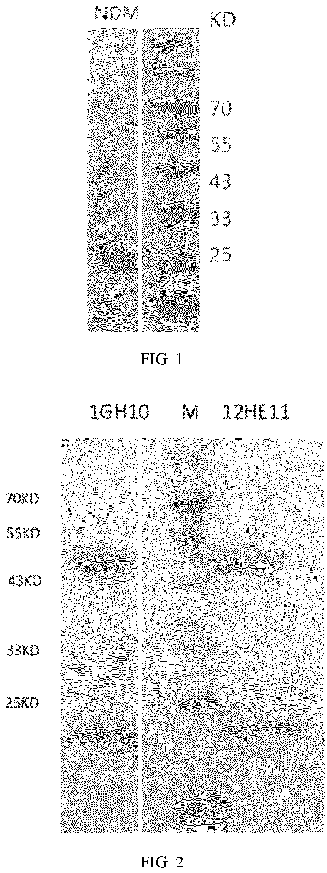

Purification: The supernatant was allowed to pass through a nickel column for purification and then dialyzed into PBS to obtain a target protein with a high purity, which had a molecular weight consistent with the predicted 28.25 kD. Sodium dodecyl sulfate-polyacrylamide gel electrophoresis (SDS-PAGE) results were shown in FIG. 1. The target protein was quantified by a bicinchoninic acid (BCA) method and then dispensed for subsequent experiments.

### 1.2 Mouse immunization

Female BALB/c mice at an age of about six weeks were immunized with the purified NDM-type carbapenemase for antibody preparation, with a protein content of 0.1 mg/mL. According to an immunization dose, the mice were divided into two groups, with 5 mice in each group. According to the protein content, an immunization dose for a first group was 25 µg/mouse, and an immunization dose for a second group was 50 µg/mouse. For the first immunization, an appropriate amount of the NDM-type carbapenemase was diluted with distilled water to 300 µl, 300 µl of Freund's complete adjuvant (FCA) was added for thorough emulsification, and a resulting emulsion was subcutaneously injected into the mice at multiple points for immunization. Two weeks later, the second immunization was carried out with the same dose, where the mice were immunized through intraperitoneal injection. Two weeks later, the mice were immunized once again through intraperitoneal injection. Seven days later, blood was collected from the mouse tail, and the enzyme-linked immunosorbent assay (ELISA) was used to measure the mouse serum titer.

A specific procedure was as follows: an ELISA plate was coated overnight at 4°C with 100 µL of 0.2 µg/mL NDM per well, then spin-dried, washed 3 times with phosphate buffered saline with tween-20 (PBST), and blocked at 37°C for 2 h with 200 µL of 5% skimmed milk powder per well. Blood was collected from the mouse tail and centrifuged at 3,000 r/min, and resulting serum was collected and doubling diluted with PBS from 1:1,000 to 1:512,000 for later use. A resulting ELISA plate was spin-dried and washed 3 times with PBST. A primary antibody diluted with PBS was added to the ELISA plate from 1:1,000 at 100 µL/well, and then the ELISA plate was incubated at 37°C for 1 h, spin-dried, and washed 3 times with PBST. A goat anti-mouse secondary antibody diluted with PBS at 1:6,000 was added to the ELISA plate at 100 µL/well, and then the ELISA plate was incubated at 37°C for 45 minutes, spin-dried, and washed 5 times with PBST. 3,3',5,5'-tetramethylbenzidine (TMB) was added to the ELISA plate at 100 µL/well, a chromogenic reaction was carried out at 37°C for 10 min, the reaction was terminated, and a value was read.

### 1.3 Cell fusion

Three days before fusion, the mice each were subjected to booster immunization with the same immunization dose as the previous immunization, where no adjuvant was added, and the booster immunization was conducted through intraperitoneal injection. One day before fusion, feeder layer cells were prepared, and one BALB/c mouse at an age of 6 to 8 weeks was taken and killed by cervical dislocation after eyeball exsanguination. The dead BALB/c mouse was sterilized in 75% alcohol for 5 minutes and fixed on a plate, and the abdominal skin was cut aseptically on an ultra-clean table. Ten mL of a hypoxanthine-aminopterin-thymidine (HAT) selection medium was drawn by an aseptic syringe and injected into the abdominal cavity of the dead BALB/c mouse, then the abdomen was gently rubbed with alcohol, cotton balls, and the medium was withdrawn and added to 40 mL of an HAT medium. A resulting mixed medium was added to four 96-well cell culture plates at 100 µL/well and incubated in a 5% CO₂ cell incubator at 37°C. One week before fusion, myeloma cells (Sp2/0 cells) were resuscitated and cultured with a 10% fetal bovine serum (FBS)-containing PRMI-1640 medium in a 5% CO₂ incubator at 37°C. Myeloma cells in a logarithmic growth stage were collected into a centrifuge tube, counted, and diluted to 10⁷ cells/mL for later use. Eyeballs were removed from the BALB/c mouse that had undergone booster immunization three days ago, and blood was collected to prepare positive serum. The mouse was killed by cervical dislocation, sterilized in 75% alcohol for 5 minutes, and placed on the ultra-clean table. Then, a spleen was collected aseptically and washed several times in a sterile plate and a connective tissue was stripped. A resulting spleen was placed on a microporous copper net and a fresh RPMI-1640 medium was added. The medium was drawn by a syringe and injected into the spleen through an end to blow spleen cells down, the operation was repeated several times, and the remaining spleen was gently ground with an inner plug of the syringe until there was no obvious red tissue mass. A spleen cell suspension in the plate was gently pipetted up and down, then transferred to a 50 mL centrifuge tube, centrifuged at 1,000 r/min for 5 minutes, and resulting spleen cells were collected and counted for later use. The spleen cells of the immunized mouse and the Sp2/0 cells were mixed at a cell number ratio of 10:1, added to a 50 mL centrifuge tube, and centrifuged at 1,000 r/min for 5 minutes, a resulting supernatant was discarded and the centrifuge tube was gently rubbed in the palm to make the two cells fully mixed. The centrifuge tube was placed in a 100 mL blue-capped bottle with 37°C hot water. 1 mL of preheated dimethyl sulfoxide/polyethylene glycol (DMSO/PEG) was added dropwise to a fusion tube within 1 minute, where the DMSO/PEG was added slowly first and then quickly, and the centrifuge tube was gently rotated during the addition of the preheated DMSO/PEG. Then, the reaction was terminated by immediately adding an penicillin-streptomycin-free and serum-free RPMI-1640 medium at an amount of 1 mL for the first minute, 2 mL for the second minute, 3 mL for the third minute, and 4 mL for the fourth minute. A resulting mixture was incubated for 5 minutes in a water bath at 37°C and then centrifuged at 800 r/min for 5 minutes, a resulting supernatant was discarded, and a resulting precipitate was suspended by HAT. A resulting suspension was thoroughly mixed with 40 mL of a 20% FBS-containing HAT selection medium preheated at 37°C, and a resulting mixture was added at 100 µL/well to 96-well cell culture plates with feeder layer cells and then incubated in a 5% CO₂ incubator at 37°C. Seven days later, a fresh HAT medium was used to replace half of the medium in each of the culture plates. Ten days later, a hypoxanthine-thymidine (HT) medium was used to replace all of the medium in each of the culture plates. Positive cells in the 96-well cell culture plates were subcloned by a limiting dilution method: feeder layer cells were first prepared according to the above method, and hybridoma cells to be cloned were counted, diluted with an HT medium to 5 to 8 cells/mL, and added at 100 µL/well to 96-well cell culture plates with feeder layer cells. Each hybridoma cell line was cloned into one 96-well cell culture plate and cultured in a 5% CO₂ cell incubator at 37°C. About 5 days later, a number of clones in a cell well was counted and the clones were labeled. On day 7, the medium was replaced with a new medium. The detection was carried out when 1/3 to 1/2 of a well bottom was covered by the cells. When all cell wells in the 96-well plates were positive after 2 to 3 times of cloning, an expanded culture could be carried out, and resulting cell lines were fixed and frozen. The hybridoma cells that were tested as positive were subjected to expanded culture and then frozen. A specific process was as follows: hybridoma cells in a vigorous growth state were gently blown off from the cell bottle with a penicillin-streptomycin-free and serum-free Dulbecco's modified eagle medium (DMEM) and centrifuged at 1,000 r/min for 5 minutes. A resulting supernatant was discarded, a freezing medium (including 40% of an RPMI-1640 medium, 50% of FBS, and 10% of DMSO) was added, and a resulting mixture was thoroughly mixed and dispensed in freezing tubes. The freezing tubes were placed in a freezing box and then stored in a - 70°C refrigerator. One day later, the freezing tubes were transferred to liquid nitrogen and recorded.

### 1.4 Preparation of ascites

Female BALB/c mice at an age of 10 to 12 weeks were intraperitoneally injected with sterile liquid paraffin at a dose of 0.5 mL/mouse, and 7 days later, the mice each were intraperitoneally injected with hybridoma cells in the logarithmic stage at a dose of 5 × 10⁶ cells/mouse. The mice were observed every day. About 7 to 10 days later, when the abdomens of the mice noticeably swelled, the lower abdominal skin was disinfected with 75% alcohol cotton balls, and a 16-gauge needle was used to penetrate the abdominal cavity to collect ascites. After the ascites was regenerated and accumulated, the ascites was collected again. The collected ascites was centrifuged at 3,000 r/min for 10 minutes, and a middle clear layer was taken, filtered through a filter paper, dispensed, and stored at -70°C.

### 1.5 Antibody purification

Ascites was purified using a Protein-G column through the following steps: 2 mL (n) of ascites was taken and centrifuged at 10,000 g, a clear layer was taken and added to 2 mL of a wash buffer (1:1), and a resulting mixture was thoroughly mixed. The column was rinsed with 20% ethanol and equilibrated with 8 mL of a wash buffer. A sample was allowed to pass through the column at a flow rate of 8 S/drop. The sample was repeatedly loaded 3 times, and a resulting precipitate was washed with 15 mL of a wash buffer at a flow rate of 8 S/drop. After the washing was completed, elution was conducted with 10 mL of an elution buffer. After the elution was completed, an eluate was adjusted to a pH of 7.4 using 1 M Tris with a pH of 9, then concentrated with a concentrating column, and dialyzed overnight at 4°C in a 50 kd dialysis bag filled with PBS.

### Embodiment 2: Identification of an anti-NDM-type carbapenemase mouse mAb

### 2.1 Identification of antibody subtypes

According to the instructions of the SIGMA kit, a subtype of a mAb was identified by an ELISA capture method as follows: A mAb subtype identification reagent was diluted at 1:1,000 and added to wells of an ELISA plate at a dose of 100 µL/well. The ELISA plate was incubated at 37°C for 1 h, washed 3 times with PBST, and pat-dried. An antibody was diluted at 1:1,000 and added at a dose of 100 µL/well, and the ELISA plate was incubated at 37°C for 1 h, washed 3 times with PBST, and pat-dried. A goat anti-mouse immunoglobulin G-horseradish peroxidase (IgG-HRP) was diluted at 1:6,000 and added at a dose of 100 µL/well, and the ELISA plate was incubated at room temperature for 30 minutes. A chromogenic reaction was carried out for 10 minutes to 20 minutes. The subtype of the mAb was determined as a subtype of a subtype reagent added to a well with the maximum OD₄₅₀ value. The antibody 12HE11 and the antibody 1GH10 each had an antibody subtype of immunoglobulin G1 (IgG1).

### 2.2 Determination of an antibody titer

A titer of a purified antibody was determined by an indirect ELISA method through the following steps: The NDM carbapenemase was diluted to 0.2 µg/mL and added to an ELISA plate at a dose of 100 µL/well, and a non-coated control was set. The ELISA plate was coated at 4°C overnight, spin-dried, and washed 3 times with PBST; and then the ELISA plate was blocked at 37°C for 2 h with 5% skimmed milk powder at a dose of 200 µL/well, spin-dried, and washed 3 times with PBST. An antibody (initial concentration: 1 mg/mL) was doubling diluted from 1:1,000 to obtain 12 concentrations and each concentration was added to the ELISA plate at a dose of 100 µL/well; and a non-coated control was set. The ELISA plate was incubated at 37°C for 1 h, spin-dried, and washed 3 times with PBST. A goat anti-mouse secondary antibody diluted with PBS at 1:6,000 was added at a dose of 100 µL/well, and then the ELISA plate was incubated at 37°C for 45 minutes, spin-dried, and washed 5 times with PBST. TMB was added at a dose of 100 µL/well, and a chromogenic reaction was carried out at 37°C for 10 minutes. The reaction was terminated, and a value was read. After purification, the antibody was diluted to 1 mg/mL, and a titer of the antibody reached 1: 1,280,000 or more.

### 2.3 Identification of a purity and molecular weight of an antibody

SDS-PAGE was used to identify the molecular weight and purity of the antibody. For gel preparation, a concentration of a separated gel was 12%, and a concentration of a concentrated gel was 5%. For sample preparation, 20 µL of a sample and 20 µL of a buffer were thoroughly mixed and boiled for 3 minutes. Each well was loaded with 20 µL of the sample and a protein pre-staining marker control was set. Electrophoresis was carried out at 80 V for 30 minutes and at 120 V for 2 h. After the electrophoresis was completed, the sample was stained with a Coomassie brilliant blue (CBB) solution. Decolorization was carried out by boiling in deionized water for 5 minutes each time with 3 times in total. A molecular weight of a heavy chain of the IgG antibody is generally 50 kDa to 75 kDa and a molecular weight of a light chain of the IgG antibody is about 25 kDa. The purified mAb was identified by SDS-PAGE. As shown in FIG. 2, the antibody 1GH10 and the antibody 12HE11 have clear bands at 50 kDa to 75 kDa and about 25 kDa, respectively.

### Embodiment 3: Genetic validation of an anti-NDM-type carbapenemase mouse mAb

Ig variable region genes were cloned by a reverse transcription-polymerase chain reaction (RT-PCR) method. Total RNA was extracted, and the single-stranded complementary DNA (cDNA) was synthesized. Total RNA was extracted from each of the 12HE11 and 1GH10 hybridoma cell lines using a Trizol method (a kit purchased from Invitrogen) and reverse-transcribed into a cDNA library using Moloney murine leukemia virus reverse transcriptase (M-MLV RT) (purchased from Invitrogen):
upstream primer of the heavy chain backbone region
P1: 5'SAGGTGMAGCTKCASSARTCWGG3', as shown in SEQ ID NO: 21;
downstream primer of the heavy chain variable region
P2: 5'TGGGGSTGTYGTTTTGGCTGMRGAGACRGTGA3', as shown in SEQ ID NO: 22;
upstream primer of the light chain precursor peptide
P3: 5'ATGGATTTTCAAGTGCAGATTTTCAG3', as shown in SEQ ID NO: 23;
downstream primer of the light chain variable region
P4: 5'GGATACAGTTGGTGCAGCATCAGCCCGTTT3', as shown in SEQ ID NO: 24;
A PCR reaction system (50 µL) was prepared as follows:
   cDNA: 2 µL; upstream primer (10 µM): 2 µL; downstream primer (10 µM): 2 µL; dNTP mixture: 2 µL; pyrococcus furiosus (pfu) DNA polymerase (5 U/µL): 1 µL; 10×pfu Buffer II: 5 µL; and ddH₂O: making up to 50 µL.

Reaction conditions: pre-denaturation at 95°C for 5 minutes; 35 cycles of 95°C for 30 s, 58°C for 30 s, and 72°C for 1 minute; and finally, extension at 72°C for 10 minutes.

Heavy chain variable region (VH) and light chain variable region (VL) fragments were separated and recovered by agarose gel electrophoresis. The recovered VL and VH fragments were ligated with a pMD19-T (simple) vector (Takara), and a 10 µL ligation system was as follows:
70 ng of each of the VL PCR product and the VH PCR product, 1 µL of the pMD19-T (simple) vector, 5 µL of a Solution I ligation reaction solution, and the balance of ddH₂O. The ligation was carried out overnight at 4°C.

A ligation product was transformed into *E.coli* DH5α competent bacteria and cultured overnight at 37°C. A single colony was selected and shaken at 37°C for 2 h, and a resulting bacterial liquid was identified by PCR, with cDNA of a corresponding antibody as a positive control. A reaction system (25 µL) was prepared as follows:
bacterial liquid: 1 µL; upstream primer (10 µM): 1 µL; downstream primer (10 µM): 1 µL; dNTP mixture (each of the dNTPs: 2.5 Mm): 2 µL; Taq DNA polymerase (5 U/µL): 0.5 µL; 10×Taq Buffer (Mg²⁺ plus): 2.5 µL; and water: making up to 25 µL. Reaction conditions were the same as previous.

PCR-positive clones were selected for expanded culture, and positive clone plasmids were extracted by a plasmid extraction kit (Takara) and sent for sequencing. At least five cloned samples were sequenced for each chain of each antibody until at least three samples had the same sequencing results. The sequences of the heavy chain variable region and the light chain variable region of the antibodies 12HE11 and 1GH10 were successfully cloned, which were consistent with the characteristics of typical antibody variable region sequences.

### Embodiment 4: Preparation of an NDM carbapenemase test card by the colloidal gold method

The NDM-type carbapenemase test card was prepared by a colloidal gold method. The double-antibody sandwich immuno-colloidal gold test card was prepared as follows:
Step 1: aA 0.1 M K₂CO₃ solution was added to a colloidal gold solution under stirring, a pH was adjusted, then the anti-NDM-type carbapenemase mAb 12HE11 was added, and a resulting mixture was stirred. A 10% bovine serum albumin (BSA) solution and 2% PEG 20000 were added, and a resulting mixture was stirred. The mixture was centrifuged at a low speed, a resulting supernatant was collected and then centrifuged at a high speed, and a resulting precipitate was collected, resuspended with colloidal gold, and diluted to a specified volume to obtain a gold-labeled antibody.
Step 2: The gold-labeled antibody was sprayed on a glass cellulose membrane and the glass cellulose membrane was dried to obtain a gold-labeled pad.
Step 3: A 1% thimerosal sodium solution was added to the anti-NDM-type carbapenemase mouse mAb 1GH10, and a resulting mixture was thoroughly mixed to obtain a test line coating solution. Then, PBS and a 1% thimerosal sodium solution were added to goat anti-mouse IgG and a resulting mixture was thoroughly mixed to obtain a quality control line coating solution. The quality control line coating solution and the test line coating solution each were streaked on a nitrocellulose membrane (NC) and the NC was dried to obtain a coated membrane.
Step 4: The coated membrane was attached to a bottom plate, then the gold-labeled pad and the absorbent paper were attached to the coated membrane, and a resulting product was laminated and then cut to obtain the NDM-type carbapenemase test card prepared by the colloidal gold method.

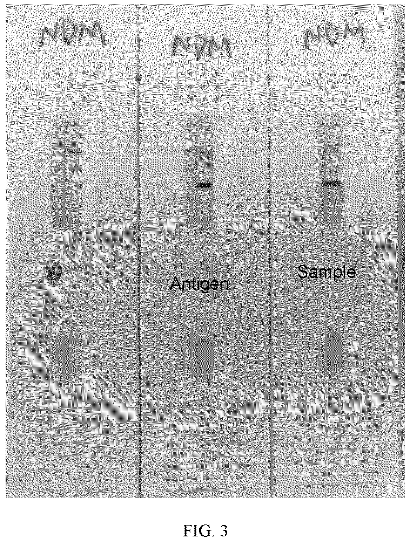

The NDM-type carbapenemase test card prepared by the colloidal gold method above was taken, and a blank sample, an NDM-type carbapenemase, and an NDM-type carbapenemase-containing positive sample each were spotted on the test card. Test results were shown in FIG. 3, where the test results were for the blank sample, the NDM-type carbapenemase, and the NDM-type carbapenemase-containing positive sample from left to right, respectively. It can be seen that a test result of the blank sample is negative and test results of the NDM-type carbapenemase and the NDM-type carbapenemase-containing positive sample all are positive, indicating that the NDM-type carbapenemase test card prepared by the colloidal gold method in the present disclosure can detect the NDM-type carbapenemase and the corresponding positive samples.

## Claims

1. An anti-NDM-type carbapenemase mouse mAb, **characterized in that** the anti-NDM-type carbapenemase mouse mAb is:
an antibody 1GH10 comprising a light chain variable region and a heavy chain variable region, wherein the light chain variable region comprises CDRL1 as shown in SEQ ID NO: 1, CDRL2 as shown in SEQ ID NO: 2, and CDRL3 as shown in SEQ ID NO: 3 and the heavy chain variable region comprises CDRH1 as shown in SEQ ID NO: 4, CDRH2 as shown in SEQ ID NO: 5, and CDRH3 as shown in SEQ ID NO: 6; the light chain variable region of the antibody 1GH10 has an amino acid sequence as shown in SEQ ID NO: 7, and the heavy chain variable region of the antibody 1GH10 has an amino acid sequence as shown in SEQ ID NO: 9;
or
an antibody 12HE11 comprising a light chain variable region and a heavy chain variable region, wherein the light chain variable region comprises CDRL1 as shown in SEQ ID NO: 11, CDRL2 as shown in SEQ ID NO: 12, and CDRL3 as shown in SEQ ID NO: 13 and the heavy chain variable region comprises CDRH1 as shown in SEQ ID NO: 14, CDRH2 as shown in SEQ ID NO: 15, and CDRH3 as shown in SEQ ID NO: 16; the light chain variable region of the antibody 12HE11 has an amino acid sequence as shown in SEQ ID NO: 17, and the heavy chain variable region of the antibody 12HE11 has an amino acid sequence as shown in SEQ ID NO: 19.

2. The anti-NDM-type carbapenemase mouse mAb according to claim 1, **characterized in that** the antibody 1GH10 is produced by an anti-NDM-type carbapenemase mouse hybridoma cell line with an accession number of CGMCC No. 21963; and
the antibody 12HE11 is produced by an anti-NDM-type carbapenemase mouse hybridoma cell line with an accession number of CGMCC No. 21964.

3. A nucleic acid molecule, comprising a nucleotide encoding the anti-NDM-type carbapenemase mouse mAb according to claim 1.

4. The nucleic acid molecule according to claim 3, **characterized in that** a nucleotide sequence of the nucleic acid molecule encoding the light chain variable region of the antibody 1GH10 is shown in SEQ ID NO: 8, and a nucleotide sequence of the nucleic acid molecule encoding the heavy chain variable region of the antibody 1GH10 is shown in SEQ ID NO: 10; and
a nucleotide sequence of the nucleic acid molecule encoding the light chain variable region of the antibody 12HE11 is shown in SEQ ID NO: 18, and a nucleotide sequence of the nucleic acid molecule encoding the heavy chain variable region of the antibody 12HE11 is shown in SEQ ID NO: 20.

5. An application of the anti-NDM-type carbapenemase mouse mAb according to any one of claims 1 to 2 in preparation of a reagent for *in vitro* detection of an NDM-type carbapenemase antigen.

6. The application of the anti-NDM-type carbapenemase mouse mAb in preparation of a reagent for detecting an NDM-type carbapenemase antigen according to claim 5, **characterized in that** the anti-NDM-type carbapenemase mouse mAb is used to prepare an *in vitro* diagnostic kit or a microfluidic chip; and the *in vitro* diagnostic kit is a colloidal gold immunoassay kit, a chemiluminescence kit, a radioimmunoassay kit, an enzyme-linked immunoassay kit, or a fluorescence immunoassay kit.

7. The application of the anti-NDM-type carbapenemase mouse mAb in preparation of a reagent for detecting an NDM-type carbapenemase antigen according to claim 5, **characterized in that** when the anti-NDM-type carbapenemase mouse mAb is used in preparation of a double-antibody sandwich immuno-colloidal gold test card, the antibody 1GH10 is used as a coated antibody and the antibody 12HE11 is used as a gold-labeled antibody; or
the antibody 12HE11 is used as a coated antibody and the antibody 1GH10 is used as a gold-labeled antibody.

## Patentansprüche

1. Ein Anti-NDM-Typ-Carbapenemase-Maus-mAb, **dadurch gekennzeichnet, dass** das Anti-NDM-Typ-Carbapenemase-Maus-mAb ist:
ein Antikörper 1GH10, der eine leichte Ketten-Variable-Region und eine schwere Ketten-Variable-Region umfasst, wobei die leichte Ketten-Variable-Region CDRL1, gezeigt in SEQ ID NO: 1, CDRL2, gezeigt in SEQ ID NO: 2, und CDRL3, gezeigt in SEQ ID NO: 3 umfasst und die schwere Ketten-Variable-Region CDRH1, gezeigt in SEQ ID NO: 4, CDRH2, gezeigt in SEQ ID NO: 5, und CDRH3, gezeigt in SEQ ID NO: 6, umfasst; die leichte Ketten-Variable-Region des Antikörpers 1GH10 weist eine Aminosäuresequenz auf, gezeigt in SEQ ID NO: 7, und die schwere Ketten-Variable-Region des Antikörpers 1GH10 weist eine Aminosäuresequenz auf, gezeigt in SEQ ID NO: 9;
oder
ein Antikörper 12HE11, der eine leichte Ketten-Variable-Region und eine schwere Ketten-Variable-Region umfasst, wobei die leichte Ketten-Variable-Region CDRL1, gezeigt in SEQ ID NO: 11, CDRL2, gezeigt in SEQ ID NO: 12, und CDRL3, gezeigt in SEQ ID NO: 13 umfasst und die schwere Ketten-Variable-Region CDRH1, gezeigt in SEQ ID NO: 14, CDRH2, gezeigt in SEQ ID NO: 15, und CDRH3, gezeigt in SEQ ID NO: 16, umfasst; die leichte Ketten-Variable-Region des Antikörpers 12HE11 weist eine Aminosäuresequenz auf, gezeigt in SEQ ID NO: 17, und die schwere Ketten-Variable-Region des Antikörpers 12HE11 weist eine Aminosäuresequenz auf, gezeigt in SEQ ID NO: 19.

2. Das Anti-NDM-Typ-Carbapenemase-Maus-mAb nach Anspruch 1, **dadurch gekennzeichnet, dass** der Antikörper 1GH10 durch eine Anti-NDM-Typ-Carbapenemase-Maus-Hybridomzelllinie mit der Hinterlegungsnummer CGMCC No. 21963 produziert wird; und
der Antikörper 12HE11 durch eine Anti-NDM-Typ-Carbapenemase-Maus-Hybridomzelllinie mit der Hinterlegungsnummer CGMCC No. 21964 produziert wird.

3. Ein Nukleinsäuremolekül, umfassend ein Nukleotid, das das Anti-NDM-Typ-Carbapenemase-Maus-mAb nach Anspruch 1 kodiert.

4. Das Nukleinsäuremolekül nach Anspruch 3, **dadurch gekennzeichnet, dass** eine Nukleotidsequenz des Nukleinsäuremoleküls, das die leichte Ketten-Variable-Region des Antikörpers 1GH10 kodiert, in SEQ ID NO: 8 gezeigt ist, und eine Nukleotidsequenz des Nukleinsäuremoleküls, das die schwere Ketten-Variable-Region des Antikörpers 1GH10 kodiert, in SEQ ID NO: 10 gezeigt ist; und
eine Nukleotidsequenz des Nukleinsäuremoleküls, das die leichte Ketten-Variable-Region des Antikörpers 12HE11 kodiert, ist in SEQ ID NO: 18 gezeigt ist, und eine Nukleotidsequenz des Nukleinsäuremoleküls, das die schwere Ketten-Variable-Region des Antikörpers 12HE11 kodiert, in SEQ ID NO: 20.

5. Eine Verwendung des Anti-NDM-Typ-Carbapenemase-Maus-mAb nach einem der Ansprüche 1 bis 2 bei der Herstellung eines Reagenzes zur in-vitro-Detektion eines NDM-Typ-Carbapenemase-Antigens.

6. Verwendung des Anti-NDM-Typ-Carbapenemase-Maus-mAb bei der Herstellung eines Reagenzes zur Detektion eines NDM-Typ-Carbapenemase-Antigens nach Anspruch 5, **dadurch gekennzeichnet, dass** das Anti-NDM-Typ-Carbapenemase-Maus-mAb zur Herstellung eines in-vitro-Diagnostikumsatzes oder eines mikrofluidischen Chips verwendet wird; und das in-vitro-Diagnostikumsatz ist ein kolloidaler Gold-Immunoassay-Satz, ein Chemilumineszenzsatz, ein Radioimmunoassay-Satz, ein Enzyme-linked-Immunoassay-Satz oder ein Fluoreszenz-Immunoassay-Satz.

7. Verwendung des Anti-NDM-Typ-Carbapenemase-Maus-mAb bei der Herstellung eines Reagenzes zur Detektion eines NDM-Typ-Carbapenemase-Antigens nach Anspruch 5, **dadurch gekennzeichnet, dass**, wenn das Anti-NDM-Typ-Carbapenemase-Maus-mAb bei der Herstellung einer Doppelantikörper-Sandwich-Immuno-Kolloidgold-Testkarte verwendet wird, der Antikörper 1GH10 als Beschichtungsantikörper verwendet wird und der Antikörper 12HE11 als goldmarkierter Antikörper verwendet wird; oder
der Antikörper 12HE11 als Beschichtungsantikörper verwendet wird und der Antikörper 1GH10 als goldmarkierter Antikörper verwendet wird.

## Revendications

1. Anticorps monoclonal de carbapénémase de type anti-NDM de souris, **caractérisé en ce que** l'anticorps monoclonal de carbapénémase de type anti-NDM de souris est :
un anticorps 1GH10 comprenant une région variable de chaîne légère et une région variable de chaîne lourde, dans lequel la région variable de chaîne légère comprend CDRL1 telle que représentée dans SEQ ID NO: 1, CDRL2 telle que représentée dans SEQ ID NO: 2, et CDRL3 telle que représentée dans SEQ ID NO: 3 et la région variable de chaîne lourde comprend CDRH1 telle que représentée dans SEQ ID NO: 4, CDRH2 telle que représentée dans SEQ ID NO: 5, et CDRH3 telle que représentée dans SEQ ID NO: 6 ; la région variable de chaîne légère de l'anticorps 1GH10 présente une séquence d'acides aminés telle que représentée dans SEQ ID NO: 7, et la région variable de chaîne lourde de l'anticorps 1GH10 présente une séquence d'acides aminés telle que représentée dans SEQ ID NO: 9 ;
ou
un anticorps 12HE11 comprenant une région variable de chaîne légère et une région variable de chaîne lourde, dans lequel la région variable de chaîne légère comprend CDRL1 telle que représentée dans SEQ ID NO: 11, CDRL2 telle que représentée dans SEQ ID NO: 12, et CDRL3 telle que représentée dans SEQ ID NO: 13 et la région variable de chaîne lourde comprend CDRH1 telle que représentée dans SEQ ID NO: 14, CDRH2 telle que représentée dans SEQ ID NO: 15, et CDRH3 telle que représentée dans SEQ ID NO: 16 ; la région variable de chaîne légère de l'anticorps 12HE11 présente une séquence d'acides aminés telle que représentée dans SEQ ID NO: 17, et la région variable de chaîne lourde de l'anticorps 12HE11 présente une séquence d'acides aminés telle que représentée dans SEQ ID NO: 19.

2. Anticorps monoclonal de carbapénémase de type anti-NDM de souris selon la revendication 1, **caractérisé en ce que** l'anticorps 1GH10 est produit par une lignée cellulaire d'hybridome de carbapénémase de type anti-NDM de souris ayant un numéro d'accès CGMCC n° 21963 ; et
l'anticorps 12HE11 est produit par une lignée cellulaire d'hybridome de carbapénémase de type anti-NDM de souris avec un numéro d'accès CGMCC n° 21964.

3. Molécule d'acide nucléique, comprenant un nucléotide codant pour l'anticorps monoclonal de carbapénémase de type anti-NDM de souris selon la revendication 1.

4. Molécule d'acide nucléique selon la revendication 3, **caractérisée en ce qu'**une séquence nucléotidique de la molécule d'acide nucléique codant pour la région variable de chaîne légère de l'anticorps 1GH10 est représentée dans SEQ ID NO: 8, et une séquence nucléotidique de la molécule d'acide nucléique codant pour la région variable de chaîne lourde de l'anticorps 1GH10 est représentée dans SEQ ID NO: 10 ; et
une séquence nucléotidique de la molécule d'acide nucléique codant pour la région variable de chaîne légère de l'anticorps 12HE11 est représentée dans SEQ ID NO: 18, et une séquence nucléotidique de la molécule d'acide nucléique codant pour la région variable de chaîne lourde de l'anticorps 12HE11 est représentée dans SEQ ID NO: 20.

5. Application de l'anticorps monoclonal de carbapénémase de type anti-NDM de souris selon l'une quelconque des revendications 1 à 2 dans la préparation d'un réactif pour la détection *in vitro* d'un antigène de carbapénémase de type NDM.

6. Application de l'anticorps monoclonal de carbapénémase de type anti-NDM de souris dans la préparation d'un réactif pour la détection d'un antigène de carbapénémase de type NDM selon la revendication 5, **caractérisée en ce que** l'anticorps monoclonal de carbapénémase de type anti-NDM de souris est utilisé pour préparer *in vitro* un kit de diagnostic ou une puce microfluidique ; et le kit de diagnostic *in vitro* est un kit de dosage immunologique d'or colloïdal, un kit de chimioluminescence, un kit de dosage radioimmunologique, un kit de dosage immunologique lié à une enzyme, ou un kit de dosage immunologique par fluorescence.

7. Application de l'anticorps monoclonal de carbapénémase de type anti-NDM de souris dans la préparation d'un réactif pour la détection d'un antigène de carbapénémase de type NDM selon la revendication 5, **caractérisée en ce que**, lorsque l'anticorps monoclonal de carbapénémase de type anti-NDM de souris est utilisé dans la préparation d'une carte de test d'or immuno-colloïdal sandwich à double anticorps, l'anticorps 1GH10 est utilisé comme anticorps revêtu et l'anticorps 12HE11 est utilisé comme anticorps marqué à l'or ; ou
l'anticorps 12HE11 est utilisé comme anticorps revêtu et l'anticorps 1GH10 est utilisé comme anticorps marqué à l'or.
